# EUROPÄISCHE PATENTSCHRIFT

(11) **EP 1 529 485 B1**
(45) Veröffentlichungstag und Bekanntmachung des Hinweises auf die Patenterteilung: **13.02.2008**
(21) Anmeldenummer: 04022621.9
(22) Anmeldetag: 23.09.2004
(51) Int. Cl.: A61B 1/24, A61B 5/00

(54) **Intraorale Kameraeinrichtung sowie Verfahren zum Erzeugen eines ausgerichteten Bildes eines intraoralen Gegenstandes, insbesondere eines Patientenzahnes**
Intraoral camera device and method to produce an aligned image of an intraoral object, preferably of a patient tooth
Dispositif et procédé de caméra intrabuccale pour la génération d'une image alignee d'un objet intrabuccal, en particulier de dents d'un patient

(30) Priorität: 10.11.2003 DE 10352394
(43) Veröffentlichungstag der Anmeldung: 11.05.2005
(73) Patentinhaber: Firma Ivoclar Vivadent AG, 9494 Schaan (LI)
(72) Erfinder: Kerschbaumer, Harald, 6833 Klaus (AT); Rohner, Gottfried, 9450 Altstätten (CH); Pokorny, Walter, 6719 Gais (AT)
(74) Vertreter: Baronetzky, Klaus

(56) Entgegenhaltungen:
- EP-A- 0 968 687
- EP-A- 1 252 858
- WO-A-01/41632
- US-A- 4 837 732
- US-A- 5 926 262
- US-A- 6 007 332
- US-A- 6 132 211
- US-A1- 2003 049 585

## Beschreibung

Die vorliegende Erfindung betrifft eine intraorale Kameraeinrichtung gemäß dem Oberbegriff von Anspruch 1 sowie ein Verfahren gemäß dem Oberbegriff von Anspruch 12.

Der Einsatz intraoraler Video- und/oder Bildaufnahmesysteme (nachfolgend als "intraorales Kamerasystem" und als Kameraeinrichtung bezeichnet), hat im Bereich der Zahnmedizin über die letzten Jahre hinweg stark zugenommen. Derartige Systeme werden in einer Vielzahl von Umgebungen eingesetzt, auch in Situationen, in denen der Zahnarzt bestimmte Merkmale eines Patientenmundes zeigen und veranschaulichen will. Diese intraoralen Kamerasysteme werden immer häufiger zum Schlüssel für komplexe Diagnose- und Behandlungsplanung. Die Forschung hat gezeigt, dass ungefähr 30% der praktizierenden Zahnärzte in der Altersgruppe zwischen 35 und 54 Jahren intraorale Kamerasysteme besitzen und verwenden. Es wird erwartet, dass dieser Prozentsatz mit zunehmender Vertrautheit mit solchen Systemen ansteigen wird, wie es aus dem Dental Procedures Report, Seite 22 bis 24, Februar 1995, ersichlich ist.

Intraorale Kamerasysteme werden oft bei Zahnrestaurationsverfahren eingesetzt. Viele Personen entscheiden sich heute für klinische Eingriffe, um ihr Lächeln und ihr äußeres Erscheinungsbild durch restaurative Verfahren aufzuwerten. Bei den meisten dieser Verfahren ist die Veränderung der Zahnform, der Zahnstellung und/oder der Farbe des Zahnes involviert.

Ein notwendiger Schritt bei der Veränderung der Farbe eines Zahnes eines Patienten ist die Bestimmung des "Farbtons" oder "shade" des vorliegenden Zahns. Hierzu wird beispielsweise die Zahnfarbe der bestehenden Zähne der Personen, die nach einem weisseren, strahlenderen Lächeln streben, bewertet, so dass ein Vergleich zwischen vorher und nachher durchgeführt werden kann. Die Farbtonbestimmung ist sogar noch wichtiger für Personen, die Zahnersatz brauchen; es ist ein Ziel derartiger Restaurationsverfahren, ein natürliches Erscheinungsbild zu erlangen. Aus diesem Grund ist es wichtig, den Farbton des bestehenden Zahns zu kennen, so dass die neue Restauration genau an ihn angeglichen werden kann.

Üblicherweise wird ein Bild eines Zahns aufgenommen und in Farbton-Analysesystemen verwendet, um geeignete Informationen über Farbton und Form zu erhalten. Bediener derartiger System finden es oft schwierig, die intraorale Kamera richtig auszurichten. Eine richtige axiale Ausrichtung und Zieleinstellung ist jedoch äußerst wichtig für eine Software-Analyse, die dann für das Bild durchgeführt wird, um den Farbton oder "shade" der bestehenden Zähe zu erfassen.

Eine derartige Dentalkamera wird in der Patentanmeldung EP 1 252 858 A2 beschrieben.

Die Größe des interessierenden Bereichs, die Kameragröße, die intraorale Stelle des interessierenden Bereichs, sowie andere Faktoren erschweren die zutreffende Analyse, da sie die zweidimensionale Aufnahme beeinflussen. Hierdurch können auch Bilder entstehen, bei denen ein Ausschnitt des betreffenden Zahns abgeschnitten ist, oder Bilder, die nicht auf den interessierenden Zahn abgestimmt sind.

Ein weiterer Nachteil bei Systemen, die frei in der Hand gehaltene Kameras verwenden, liegt darin, dass sogar geringfügige Handbewegungen (z.B. sich bewegende Finger beim Drücken des Auslösers) erhebliche Veränderungen bei der Zielausrichtung der Kamera verursachen können.

Häufig erfordert daher die Aufnahme eines zufriedenstellenden Bildes eines interessierenden intraoralen Gegenstands bei der Verwendung der bekannten Systeme wiederholte Versuche und/oder eine umständliche Bedienung verschiedener Einrichtungen.

Daher liegt der Erfindung die Aufgabe zugrunde, eine intraorale Kamereinrichtung gemäß dem Oberbegriff von Anspruch 1 sowie ein Verfahren zum Erzeugen eines axial ausgerichteten Bildes eines intraoralen Gegenstandes gemäß dem Oberbegriff von Anspruch 12 zu schaffen, die eine rasche Aufnahme ermöglichen, wobei eine umständliche Bedienung vermieden werden soll, ohne dass die Qualität der Aufnahme leidet.

Diese Aufgabe wird erfindungsgemäß durch die Ansprüche 1 und 12 gelöst. Vorteilhafte Weiterbildungen ergeben sich aus den Unteransprüchen.

Erfindungsgemäß stellt eine intraorale Kamera eine computergestützte Zieleinstellung zum Aufnehmen von Bildern intraoraler Objekte bereit. Die Zieleinstellung gewährleistet, dass das Bild eines intraoralen Objekts oder eines Bereichs, beispielsweise ein Patientenzahn, in der gewünschten Größe, im gewünschten Winkel und in der gewünschten Lage aufgenommen wird. Erfahrungsgemäß werden alle relevanten Bildaufnahmeparameter vorab fixiert und festgelegt, so dass eine Fehlhandhabung ausgeschlossen wird. Die Zieleinstellungs-Einrichtungen können eine im Wesentlichen punktförmige Lichtquelle verwenden, die auf das interessierende Objekt gerichtet ist, das gerade im Visier der Kamera oder auf diese ausgerichtet ist. Bei der punktförmigen Lichtquelle kann es sich um eine Laserdiode oder eine andere Lichtquelle handeln, die einen Lichtpunkt oder einen begrenzten Lichtbereich erzeugt. Die punktförmige Lichtquelle kann auch in die Kamera integriert werden.

Unter punktförmiger Lichquelle sei hier nicht die Form der Lichtquelle selbst verstanden, sondern die Art der von der Lichtquelle abgegebenen und auf einen Beleuchtungsbereich auftreffenden Strahlung. Diese ist bevorzugt punktförmig, also im Wesentlichen kreisförmig, so dass sie auch als Lichtpunkt oder als Lichtfleck bezeichnet werden kann.

Von der punktförmigen Lichtquelle ausgesendetes Licht, das von einem interessierenden Objekt reflektiert wird, wird erfindungsgemäß bewertet, um zu bestimmen, ob eine Kamera gerade auf eine Stelle gerichtet ist, von der das gewünschte Bild aufgenommen werden soll. Zur Bewertung wird ein Bildausschnitt der Kamera verwendet. Der Bildausschnitt kann so ausgewählt sein, dass er in Form und Größe für den Lichtpunkt geeignet ist, der von der punktförmigen Lichtquelle beim Auftreffen auf ein betreffendes Objekt oder einen intessierenden Bereich erzeugt wird. Der Abstand zwischen der Kamera und ihrem Ziel ist bevorzugt ein festgelegter Abstand sein, der durch die Verwendung eines kurzen Abstandrohrs oder eines Abstandshalters bestimmt wird.

Bei einer modifizierten Ausführungsform können die Bediener mit Informationen versorgt werden, die festlegen, in welchem Abstand die Bilder aufgenommen werden sollen.

Erfindungsgemäß ist es vorgesehen, dass ein geometrisches Muster wie ein Gitter, Rasterpunkte, eine Intensitätsverteilung oder dergleichen, also ein zweidimensionales Referenzsystem, über das Bild gelegt wird, um die Größe der Winkelabweichung beim Ausrichten der Kamera zu bestimmen und zugleich Hinweise für die Korrektur zu geben. Die Korrektur kann durch eine entsprechende Signalisierung "Schwenken" in der betreffenden Richtung realisiert werden. Es ist auch möglich, ein optisches oder akustisches Signal abzugeben, das anzeigt, dass die Kamera nun exakt zielausgerichtet ist.

In einer modifizierten Ausgestaltung ist es vorgesehen, den Auslöser für die Kamera nur dann freizugeben, wenn die Zielausrichtung vorgenommen ist. Es ist auch möglich, die Zielausrichtung als eine Art Triggersignal für das Auslösen zu verwenden, so dass beim Druck des Auslöseknopfes die Bildaufnahme lediglich dann realisiert wird, wenn die Zielausrichtung gegeben ist.

Unter Lichtpunkt sei hier ein begrenzter Lichtbereich verstanden. Typischerweise weist ein von einer punktförmigen Lichtquelle ausgesendeter Lichtfleck eine kreisförmige Gestalt auf, die mittig die höchste Intensität hat. Ein derartiger Lichtbereich oder Lichtfleck wird hier als Lichtpunkt bezeichnet.

Der Bildausschnitt einer Kamera, der zum Bewerten der Kameraausrichtung verwendet wird, wird in vorteilhafter Ausgestaltung zur Verwendung bei der Bewertung in Unterausschnitte. Der Inhalt der Unterausschnitte (z.B. ihre Lichteigenschaften) kann bewertet werden, um zu bestimmen, ob die Kamera im Wesentlichen zieleingestellt ist. Hierzu werden zwei oder mehr Unterabschnitte miteinander verglichen. Die Unterausschnitte können beispielsweise Gitterfelder des vorstehend erwähnten Gitters sein. Es ist auch möglich, über Intensitätsabstufungen der Lichtintensität eine Realisierung von Unterausschnitten vorzunehmen.

Zieleinstellungs-Einrichtungen, wie z.B. eine Zieleinstellungs-Software können eingesetzt werden, um anzuzeigen, wenn die Kamera im Wesentlichen zieleingestellt ist, basierend auf Lichtinformationen von dem von der punktförmigen Lichtquelle erzeugten Licht. Es kann ein hörbares oder sichtbares Zeichen bereitgestellt werden. Beispielsweise kann ein bestimmter Ausschnitt einer Anzeige einer Kamera ein grünes Licht zeigen, um ein geeignete Ausrichtung anzuzeigen. Ansprechend darauf kann ein Bild der augenblicklichen Kamerasicht aufgenommen und automatisch oder manuell gespeichert werden. Falls dies gewünscht ist, kann das System so konfiguriert sein, dass es dem Bediener ermöglicht wird, eine Auswahl zu treffen, um eine automatische Bildaufnahme zu ermöglichen.

In vorteilhafter Ausgestaltung der Erfindung wird die intraorale Kamera automatisiert, um eine schnelle, genaue und zuverlässige Bildaufnahme eines intraoralen Objekts zu liefern. Ein punktförmige Lichtquelle, beispielsweise eine Laserdiode, dient dazu, zu bestimmen, wann eine Kamera in einem intraoralen Kamerasystem die gewünschte Position für die Aufnahme eines Bildes erreicht hat.

Das intraorale Kamerasystem nimmt das Bild automatisch auf, ohne dass eine Mitwirkung eines Bedieners erforderlich ist, wenn gewisse Kriterien erfüllt sind. Hierzu wird das Bild in Bildausschnitte unterteilt, die je bewertet werden. Die Bildausschnitte weisen eine Form auf, die für den von der punktförmigen Lichtquelle erzeugten Lichtpunkt, der auf das intraorale Objekt scheint, geeignet ist. Der Ausschnitt wird bevorzugt in Unterausschnitte unterteilt, die zur Vergleichsanalyse verwendet werden können, wobei hierdurch die Zieleinstellung der intraoralen Kamera festgestellt wird.

Weitere Einzelheiten, Vorteile und Merkmale der Erfindung ergeben sich aus der nachfolgenden ausführlichen Beschreibung von Ausführungsbeispielen der Erfindung anhand der beiliegenden Zeichnungen, in welchen gleiche Bezugszeichen auf gleiche Bauteile hinweisen.

Es zeigen:
- Fig. 1: ein funktionales Blockdiagramm einer erfindungsgemäßen Ausführungsform einer intraoralen Kameraeinrichtung, wobei die Kameraeinrichtung nachstehend auch als Kameraystem bezeichnet wird;
- Fig. 2: ein funktionales Blockdiagramm der intraoralen Kameraeinrichtung gemäß Fig. 1 mit einem PC;
- Fig. 3: eine schematische Seitenansicht einer Ausführungsform einer erfindungsgemäßen intraoralen Kameraeinrichtung, unter Darstellung der Bildaufnahme- und Zieleinstellungseinrichtungen, die auf die Oberfläche eines Zahns gerichtet sind, gemäß der Ausführungsform gemäß Fig. 1 und 2;
- Fig. 4: eine schematische Seitenansicht einer weiteren Ausfühform einer erfindungsgemäßen intraoralen Kameraeinrichtung, wobei Bildaufnahme-, Zieleinstellungs- und Scharfeinstellungseinrichtungen, die auf die Oberfläche eines Zahns gerichtet sind, dargestellt sind.
- Fig. 5: ein Ablaufdiagramm der Schritte, die bei einer computergestützten Zieleinstellung vorgenommen werden, gemäß der erfindungsgemäßen Ausführungsform;
- Fig. 6: ein Ablaufdiagramm der Schritte, die bei einer Beurteilung der Zielausrichtung einer Kamera vorgenommen werden, gemäß einer erfindungsgemäßen Ausführungsform;
- Fig. 7: ein Ablaufdiagramm der Schritte, die bei einer Identifizierung eines Bildausschnitts vorgenommen werden, gemäß einer weiteren erfindungsgemäßen Ausführungsform;
- Fig. 8: eine Ansicht eines Kamerabildes und eines Ausschnitts, die bei der Zieleinstellung der Kamera verwendet werden, gemäß einer erfindungsgemäßen Ausführungsform;
- Figs. 9 und 10: unterschiedliche Perspektivansichten der Kamera mit Laserdioden für die Zieleinstellung der Kamera entsprechend einer erfindungsgemäßen Ausführungsform; und
- Fig. 11: eine Ansicht der in den Figs. 9 und 10 gezeigten Kamera, welche ferner eine erfindungsgemäße Kamerablende als kurzes Abstandsrohr aufweist.

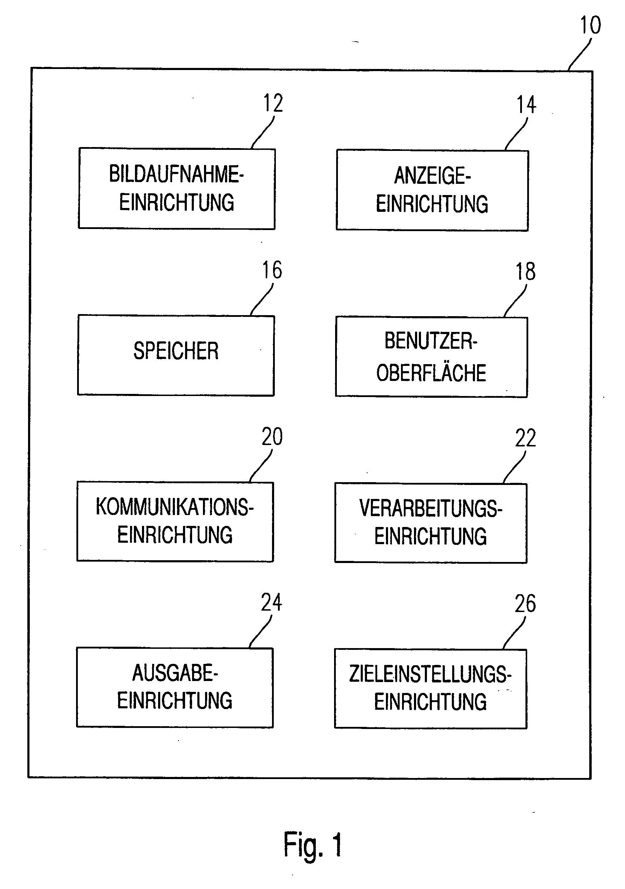

Fig. 1 zeigt ein funktionales Blockdiagramm einer ersten Ausführungsform eines intraoralen Kamerasystems. Fig. 1 zeigt ein intraorales Kamerasystem 10, das zum Erhalt von Bildinformationen eines Patientenzahns verwendet wird. Das Kamerasystem 10 kann insbesondere dazu verwendet werden, Werte für Farb- oder Formeigenschaften eines Zahns zu erzeugen. Hierdurch lässt sich der Zahn-Farbton je über den Zahn betrachtet bestimmen, und es können beliebige andere Erfassungen vorgenommen werden. In der dargestellten Ausführungsform weist das intraorale Kamerasystem 10 eine Bildaufnahmeeinrichtung 12, eine Anzeigeeinrichtung 14, einen Speicher 16, eine Benutzeroberfläche 18, eine Verarbeitungseinrichtung 22 und eine Zieleinstellungseinrichtung 26 auf.

Bei der Bildaufnahmeeinrichtung 12 handelt es sich um eine Einrichtung zum Erhalt von Informationen über physikalische und/oder farbliche Eigenschaften eines Patientenzahns. Die Bildaufnahmeeinrichtung weist eine Digitalkamera (z.B. eine intraorale Kamera) auf. Beispiele für die Bildaufnahmeeinrichtung 12 sind die Kamera Power 0/00, die von der Firma Insight in San Carlos, Kalifornien, hergestellt wird, und das von der Firma Cygnus Instruments, Inc. in Goleta, Kalifornien hergestellte Modell Cygenascope. Ein anderes Beispiel ist das Modell VistaCam ein. Bei derartigen Kameras handelt es sich um frei in der Hand gehaltene Kameras. Die Kameras weisen typischerweise eine Anzeige, einen Speicher, Software, und eine Schnittstelle für die Kommunikation mit einem PC auf. Solche Kameras sind in der Lage, die Helligkeit, die Farbe, den Farbton, die Farbart oder auch die Farbsättigung zu erfassen und hierdurch ein Bild aufzunehmen. Die Farbe kann in beliebiger geeigneter Codierung, zum Beispiel aufgeschlüsselt nach RGB, nach L*, a*, b*, oder in beliebiger anderer geeigneter Weise, erfasst werden.

In einer weiteren Ausgestaltung ist es vorgesehen, weitere Eigenschaften des Bildes oder auch von Bildausschnitten zu erfassen, beispielsweise die Fokussierungsparameter oder dergleichen. Typischerweise weist die Bildaufnahmeeinrichtung 12 eine integrierte Anzeige wie zum Beispiel eine LCD-Anzeige auf der Rückseite der digitalen Kamera auf.

Mit der Anzeigeeinrichtung 14 kann ein Bediener ein Bild eines Patientenzahns betrachten. Auch können bestimmte Softwareanalyse-Funktionen eingesetzt werden, beispielsweise zur Farbtonanalyse. Derartige erfasste Parameter können auch auf der Anzeigeeinrichtung eingespielt werden. Es ist erfindungsgemäß vorgesehen, dass die Anzeigeeinrichtung 14 jedenfalls das anzeigt, was augenblicklich im Visier der Kamera liegt, also von der Kamera erfasst wird. Anstelle der digitalen LCD-Anzeige einer Kamera kann auch ein Computermonitor eingesetzt werden, der mit der Kamera gekoppelt ist, damit die Anzeigeinformationen an den Computermonitor geliefert werden.

Der erfindungsgemäße Speicher 16 speichert typischerweise das Bild und zusätzlich bestimmte Bildparameter ab. Beispielsweise können Farbton-Leitinformationen, zum Beispiel bestimmte Farbeigenschaften von Zahnfarbtönen, gemäß einer geeigneten Farbton-Schematabelle, abgespeichert werden. Zusätzlich dient der Speicher 16 als Puffer. Er kann ein Halbleiterspeicher oder eine Festplatte oder ein beliebiger anderer Speicher, beispielsweise in einem Computer, sein. Es ist auch möglich, abgesehen von dem Bildspeicher beispielsweise in der Bildaufnahmeeinrichtung 12 einen separaten Speicher zu verwenden, wobei auch die Abspeicherung auf Medien wie einer CD oder einer DVD möglich ist.

Als Benutzeroberfläche 18 können an sich bekannte Elemente eingesetzt werden. Hierzu gehören eine Tastatur, eine Maus, ein Spracherkennungssystem und dergleichen. Die Benutzeroberfläche 18 erlaubt es dem Bediener, auf die Bildaufnahmeeinrichtung 12 in geeigneter Weise einzuwirken, zum Beispiel ein Bild aufzunehmen, die Auflösung der Bilder zu wählen, den aufzunehmenden Zahn festzulegen und dergleichen.

Die Verarbeitungseinrichtung 22 ist typischerweise ein Mikroprozessor oder ein Mikrocomputer, dessen Software der Analyse und Identifizierung von Zahnfarbtönen dient, und mit dem die erforderliche Funktion für die Bildaufnahme bereitgestellt wird. Es ist bekannt, dass die erforderlichen Elemente hierzu sowohl durch entsprechende Schaltkreise als auch durch entsprechende Software bereitgestellt werden können. Beispielsweise kann ein Computer wie ein PC für die Verarbeitungseinrichtung 22 vorgesehen sein, wobei es natürlich auch möglich ist, die ohnehin im PC vorgesehenen Elemente, wie Speicher, Anzeige, Benutzeroberfläche und Kommunikationseinrichtung sowie Ausgabeeinrichtung, für die erfindungsgemäß vorgesehenen Elemente der Anzeigeeinrichtung 14, der Benutzeroberfläche 18, des Speichers 16, der Kommunikationseinrichtung 20 und der Aufnahmeeinrichtung 22 mindestens teilweise zu verwenden. Es können separate Prozessoren oder auch Kombinationen von verschiedenen Prozessoren, die teils in der Kamera, teils im PC vorgesehen sein können, eingesetzt werden.

Erfindungsgemäß ist eine Ausrichteinrichtung oder Zieleinstellungseinrichtung 26 vorgesehen. Die Zieleinstellungseinrichtung 26 unterstützt den Bediener bei der Ausrichtung der Bildaufnahmeeinrichtung 22. Hierzu können mehrere Bauelemente in dem System 10 vorgesehen sein. Beispielsweise gehört hierzu eine punktförmige Lichtquelle, zum Beispiel eine Laserdiode, oder eine scharf fokussierte Leuchtdiode. Hierzu gehören aber auch die Hardware und die Software, die die Bildinformationen des Lichtflecks bewerten, der von der punktförmigen Lichtquelle auf dem Zahn erzeugt wird.

Die intraorale Kameraeinrichtung 10 kann Teil einer Einrichtung zur Farbton-Analyse sein. Beispiele für Farbton-Analyseeinrichtungen sind in dem US-Patent 6,305,933 B1 und in der WO 00/25696 beschrieben, wobei auf diese beiden Druckschriften vollinhaltlich Bezug genommen wird. Es ist bevorzugt, dass ein kurzes Rohr für die Nebenlicht-Abschirmung verwendet wird, so dass die aufgenommenen Bilder nicht durch Nebenlicht verfälscht werden. Ein derartiges Rohr ist beispielsweise in dem US-Patent 6,210,159 gezeigt, auf das hier ebenfalls vollinhaltlich Bezug genommen wird.

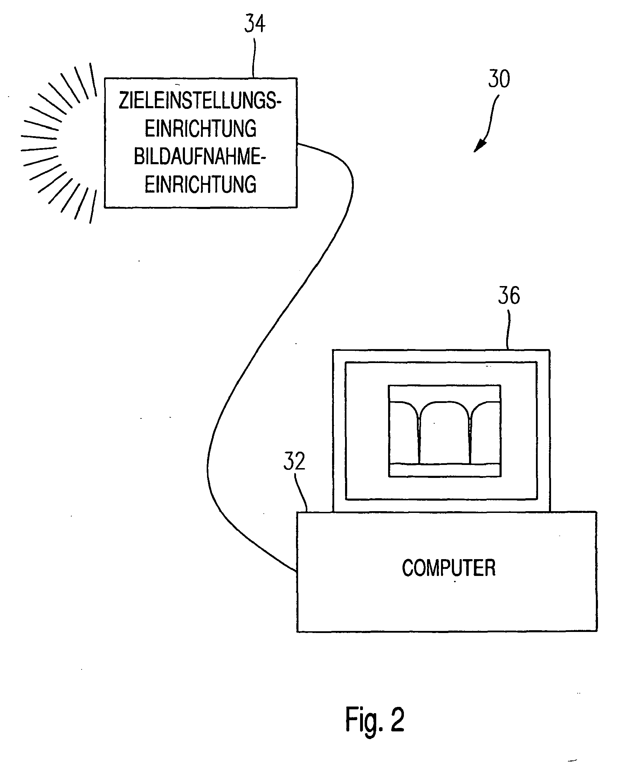

Ein Beispiel für eine intraorale Kameraeinrichtung ist aus Fig. 2 ersichtlich, wobei auch zugleich die Verbindung mit einem PC dargestellt ist. Mit der intraoralen Kameraeinrichtung 30 wird angezeigt, dass die Zielausrichtung stimmt und ein Bild des Patientenzahns aufgenommen werden kann. Die intraorale Kameraeinrichtung weist einen PC 32 und eine Bildaufnahmeeinrichtung 34 auf. In an sich bekannter Weise ist an dem PC 32 ein Computermonitor 36 als Anzeigevorrichtung angeschlossen. Die Bildaufnahmeeinrichtung 34 erfasst die Farbund Forminformationen des Patientenzahns. Auf diesem Computermonitor 36 wird angezeigt, was die Bildaufnahmeeinrichtung gerade erfasst, was also im Visier der Kamera liegt. Je nach Einstellung lässt sich auch ein abgespeichertes Bild auf dem Monitor anzeigen. Zugleich kann über dem PC 32 und/oder über die Hardware der Bildaufnahmeeinrichtung 34 die Analyse des Bildes vorgenommen werden.

Es können Analysen durchgeführt werden, um herauszufinden, ob die Bildaufnahmeeinrichtung 34 richtig auf das interessierende Objekt zieleingestellt ist. Erwünschtenfalls können auch andere Funktionen durchgeführt werden, beispielsweise die Identifizierung von Farbtönen aus einer Farbtonschematabelle. Erfindungsgemäß weist die Bildaufnahmeeinrichtung 34 die Zieleinstellungseinrichtung zur Unterstützung des Bedieners auf. Hierdurch lässt sich die Bildaufnahmeeinrichtung 34 in richtiger Weise ausrichten.

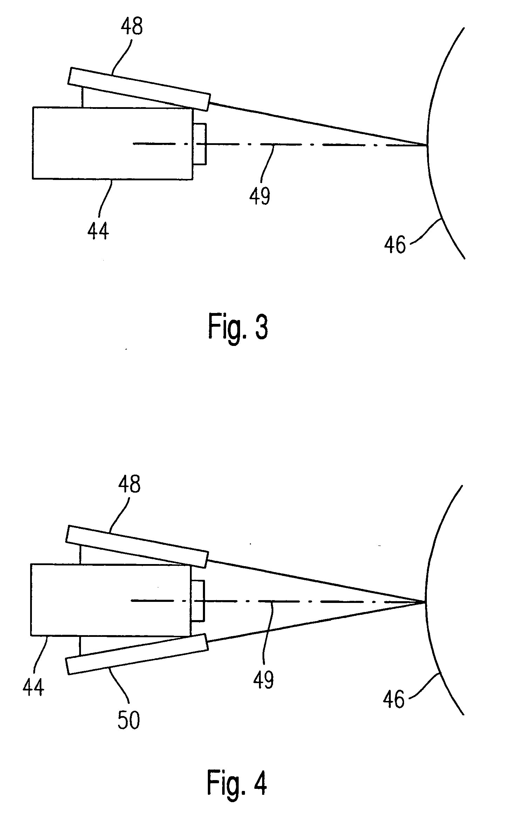

In den Figuren 3 und 4 sind schematisch geeignete Bildaufnahme- und Zieleinstellungseinrichtungen dargestellt.

Typischerweise hängt die richtige Zieleinstellung von dem Winkel ab, den eine Kamera zu einem Aufnahmeobjekt aufweist. Erfindungsgemäß wird der vorliegende Kamerawinkel analysiert, um festzulegen, ob der betreffende Winkel richtig ist oder nicht. Aus den Figuren 3 und 4 ist je eine Kamera 44 ersichtlich, die auf eine Zahnoberfläche 46 ausgerichtet ist. Die Zahnoberfläche 46 ist typischerweise konvex, wobei erläuterungshalber die Proportionen übertrieben dargestellt sind. Ein Schneidezahn weist beispielsweise im mittleren Bereich eine plane, aber strukturierte Oberfläche auf, die dann zu den Seitenflanken, also in mesialer oder distaler Richtung hin gekrümmt ist. Bei einem Ausrichtfehler einer Kamera würden sich für die betrachteten Bereiche unterschiedliche Farbtöne oder "shades" ergeben, so dass das Restaurationsergebnis verfälscht würde. Um dies zu verhindern, ist es erfindungsgemäß vorgesehen, die Zieleinstelleinrichtung 26 (Fig. 1) zu verwenden.

Hierzu wird gemäß Fig. 3 und 4 die punktförmige Lichtquelle 48 auf die Zahnoberfläche 46 ausgerichtet. Die punktförmige Lichtquelle 48 ist in dem dargestellten Ausführungsbeispiel auf der Kamera 44 montiert, und zwar so, dass sie der optischen Achse 49 eng benachbart ist, jedoch von dieser beabstandet ist.

Um diese Divergenz zu kompensieren, ist die Lichtquelle 48 in einem geringen Winkel von beispielsweise 1 bis 10° gegenüber der optischen Achse 49 auf der Kamera montiert. Die Ausrichtung ist so gewählt, dass bei einem vorgegebenen Abstand zwischen Kamera 44 und Zahnoberfläche 46 der Auftreffpunkt der optischen Achse 49 mit dem Lichtpunkt zusammenfällt, der durch die Lichtquelle 48 erzeugt wird. Es versteht sich, dass hierdurch auch eine Abstandsfestlegung erfolgt, die zugleich die Fokussierung der Bildaufnahmeeinrichtung 12 in der Kamera 44 erleichtert.

Um eine derartige Fokussierung und damit die Abstandsfestlegung auch visuell für den Bediener, beispielsweise den Zahnarzt, deutlich zu machen, ist es gemäß Fig. 4 vorgesehen, mit zwei Lichtquellen 48 und 50 zu arbeiten. Bei richtiger Abstandswahl fallen die Lichtpunkte beider Lichtquellen 48 und 50 zusammen, wobei dann zugleich dort die optische Achse 49 den erzeugten Doppel-Lichtpunkt durchtritt, und zwar bevorzugt mittig.

Es ist auch möglich, ein kurzes Rohr oder einen anderen geeigneten Abstandshalter zu verwenden, der den Abstand von vorneherein festlegt. Auch ist es möglich, eine Helligkeitsmessung über die Kamera 44 vorzunehmen, die die Helligkeit des erzeugten Lichtpunkts misst und bei maximaler Helligkeit anzeigt, dass die Zielausrichtung vorgenommen ist.

Aus Fig. 4 ist ersichtlich, dass beide Lichtquellen 48 und 50 im gleichen, jedoch zueinander spiegelsymmetrischen, Winkel zur Kamera angeordnet sind. Es versteht sich, dass anstelle dessen auch eine beliebige andere Anzahl von geeigneten Lichtquellen beispielsweise kreisförmig um die Kamera verteilt vorgesehen sein können.

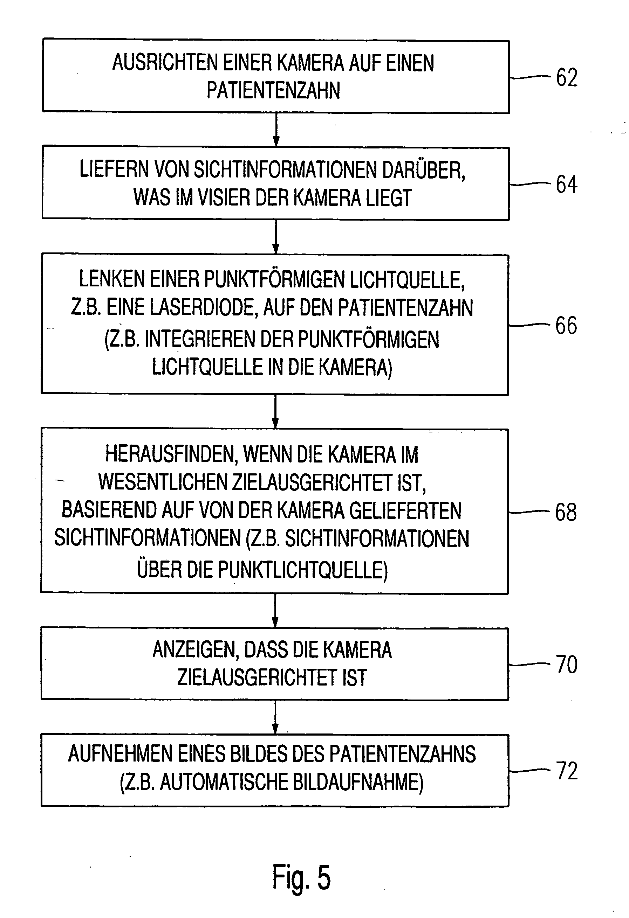

Aus Fig. 5 ist ein Schema nach der Art eines Ablaufdiagramms ersichtlich, das die für das erfindungsgemäße Verfahren erforderlichen Schritte zeigt. Gemäß dem Funktionsblock 62 wird eine Kamera auf einen Patientenzahn ausgerichtet, um ein Bild des Zahns aufzunehmen. Gemäß Schritt 64 werden Sichtinformationen darüber geliefert, was von der Kamera gerade erfasst wird. Gemäß Schritt 66 wird eine punktförmige Lichtquelle, zum Beispiel eine Laserdiode, auf den Patientenzahn gerichtet, beispielsweise die integrierte Lichtquelle 48 oder 50 gemäß Fig. 3 und Fig. 4. Es versteht sich, dass anstelle dessen grundsätzlich auch eine externe Lichtquelle auf den Patientenzahn gerichtet werden könnte.

Gemäß Schritt 36 wird über die Zieleinstellungseinrichtung 26 herausgefunden, wann die Kamera im Wesentlichen zielausgerichtet ist.

Wie es aus Schritt 70 ersichtlich ist, wird dann angezeigt, ob die Kamera zielausgerichtet ist. Hierzu kann beispielsweise eine hörbare oder sichtbare Anzeigeeinrichtung vorgesehen sein, die dem Bediener anzeigt, dass die Kamera zielausgerichtet ist. Wie es in Schritt 72 dargestellt ist, kann dann das Bild des Patientenzahns aufgenommen werden, beispielsweise automatisch, wenn in dem Schritt 70 davor für eine gewisse Zeitdauer angezeigt ist, dass die Kamera zielausgerichtet ist.

Von diesen Schritten werden die Schritte 62 und 66 typischerweise vom Benutzer vorgenommen, während die Schitte 64, 66, 70 und 72 in der bevorzugten Ausführungsform automatisch, also computergestützt, erfolgen.

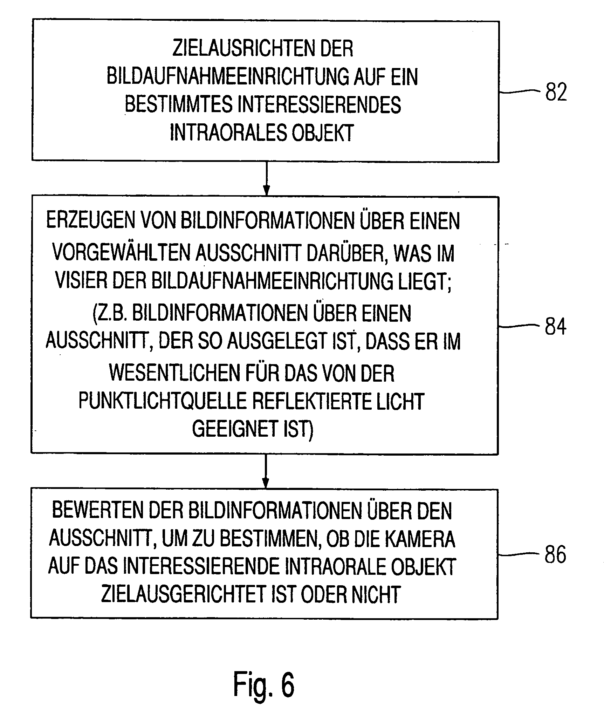

Die Zieleinstellung erfolgt bevorzugt so, dass ein begrenzter Ausschnitt des erfassten Bildes ausgewertet wird. Dies ist im Einzelnen in Fig. 6 dargestellt. In Schritt 82 ist festgehalten, dass die Bildaufnahmeeinrichtung auf ein bestimmtes interessierendes intraorales Objekt, also in der Regel den zu erfassenden Zahn, ausgerichtet wird. Die Bildinformationen werden dann - wie es in Schritt 84 festgehalten ist - erfasst, wobei ein vorgegebener oder vorgewählter Ausschnitt festgelegt ist, der einen kleinen Teil des gesamten Bildes darstellt. Die Fläche des betreffenden Ausschnitts kann beispielsweise weniger als 10 %, insbesondere weniger als 1 % des gesamten Bildes, umfassen.

Die Ausschnittgröße hängt von dem Lichtfleck und seiner Größe und Gestalt ab, der durch die Lichtquelle 48 und 50 auf dem Zahn erzeugt wird. Erfindungsgemäß ist es jedenfalls vorgesehen, dass der Ausschnitt den gesamten Lichtfleck erfasst.

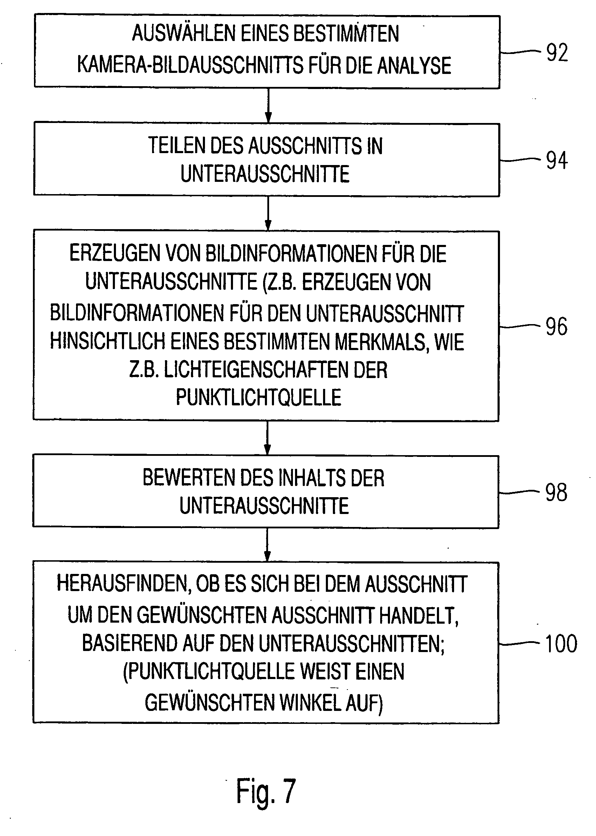

In vorteilhafter Ausgestaltung wird der Ausschnitt nun in Unterausschnitte unterteilt, wie es weiter unten anhand von Fig. 7 näher erläutert ist.

Anstelle der recht preisgünstigen Laserdiode ist es auch möglich, einen Laser zu verwenden, der einen scharf abgegrenzten Lichtfleck oder Lichtpunkt erzeugen kann. Ein Laser ist jedoch wesentlich teurer und bringt erfindungsgemäß nur einen ganz geringen Zusatznutzen, so dass preisgünstige Lichtquellen demgegenüber bevorzugt sind.

Wie es in Schritt 86 deutlich gemacht wird, werden die Bildinformationen des Ausschnittes bewertet, um zu bestimmen, ob die Kamera richtig auf das intraorale Objekt ausgerichtet ist. Beispielsweise können Bildinformationen hinsichtlich der Farbe, der Form und der Helligkeit des von der punktförmigen Lichtquelle erzeugten Lichtpunkts bewertet werden, um dies zu bestimmen.

Von den Schritten in Fig. 6 sind die Schritte 84 und 86 typischerweise Schritte, die von dem Computer vorgenommen werden, während Schritt 82 vom Bediener realisiert wird.

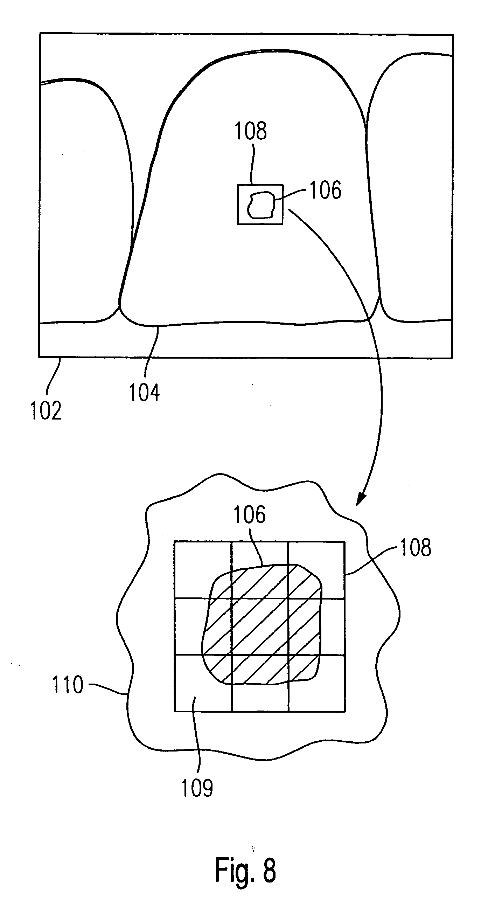

Aus Fig. 7 ist ersichtlich, in welcher Weise die Bildinformation bewertet wird. In vorteilhafter Ausgestaltung wird ein Gitter von 3 x 3 Gitterfeldern erzeugt, wie es auch in Fig. 8 dargestellt ist. Insofern wird nach dem Auswählen eines bestimmten Kamerabildausschnitts für die Analyse (Schritt 92) der Ausschnitt in Schritt 94 in Unterausschnitte geteilt. Die Festlegung kann hier automatisch erfolgen, aber gegebenenfalls auch durch eine Justierung durch den Bediener. Bevorzugt enthält jeder Unterausschnitt eine gleiche Anzahl von Pixeln, beispielsweise 20 Pixel, wobei es sich versteht, dass beliebige andere Teilungen vorgenommen werden können.

Gemäß Schritt 96 werden nun die Bildinformationen der Unterausschnitte erfasst. Hierzu gehören die Lichtparameter jedes Unterausschnitts, die dann in Schritt 98 vergleichend bewertet werden. Gemäß Schritt 100 wird dann festgelegt, ob einer der Unterausschnitte der gewünschte Ausschnitt ist, der die Mitte des Zahns anzeigt.

Auch wenn das erfindungsgemäße Verfahren hier bezogen auf eine Bilderfassung eines vollständigen Zahns beschrieben ist, versteht sich, dass anstelle dessen auch ein Teil eines Zahns erfasst und eine entsprechende Bewertung vorgenommen werden kann.

Zur Bewertung und Identifizierung der Unterausschnitte können beispielsweise Helligkeitseigenschaften von zwei Unterausschnitten verglichen werden, die einander gegenüberliegen. Die Ausrichtung wird dann als mittig festgelegt, wenn die Helligkeit dieser diametral einander gegenüberliegenden Unterausschnitte übereinstimmt. Dies funktioniert dann, wenn der von der Lichtquelle 48 und 50 erzeugte Lichtfleck eine symmetrische Helligkeitsverteilung, beispielsweise nach einer Gaußkurve, aufweist.

Es ist auch möglich, die absolute Helligkeit des zentralen Unterausschnits mit der Helligkeit eines weiter außen gelegenen Unterausschnitts zu vergleichen. In beliebiger anderer Weise kann die Symmetrie der Helligkeit beurteilt werden, wobei auch erfasst werden kann, ob die horizontal-seitlichen und die vertikal-seitlichen Unterausschnitte je die gleichen Helligkeiten aufweisen.

Aus Fig. 8 ist schematisch ersichtlich, in welcher Weise eine Kamerasicht 102, die ein Bild eines Zahns 104 aufweist, ausgewertet werden kann. Licht von einer punktförmigen Lichtquelle kann auf den Zahn 104 gerichtet werden, um einen Lichtpunkt 106 auf dem Zahn 104 zu erzeugen. Der Raster (Abschnitt 108) ist ein Ausschnitt, der zur Bewertung von Lichteigenschaften des von der punktförmigen Lichquelle erzeugten Lichts ausgewählt wird, wenn das Licht auf den Zahn 104 fällt.

Weiter unten ist in Fig. 8 eine Vergrößerung des Ausschnitts 108 und des Lichtpunkts 106 dargestellt. Das Raster (Ausschnitt 108) wird erfindungsgemäß in eine Vielzahl von Feldern (Unterausschnitte 109) aufgeteilt. Dieser Ausschnitt kann eine Form und Größe aufweisen, die so ausgewählt ist, dass sie für die typische Form und Größe des Lichtpunkts 106 geeignet ist. Eine exakte Ausrichtung ist erst dann erreicht, wenn sich der Lichtpunkt der Lichtquelle im zentralen Feld des Rasters (Ausschnitt 108) befindet. Es versteht sich, dass typische Kameraabstände eingesetzt werden und die Größe des so erzeugten Lichtpunkts empirisch ermittelt werden kann. Beispiele für intraorale Kamerasysteme sind veranschaulichend in den Druckschriften P 101 20 717.4 und P 101 26 887.4 gezeigt auf welche hierbei vollinhaltlich Bezug genommen wird.

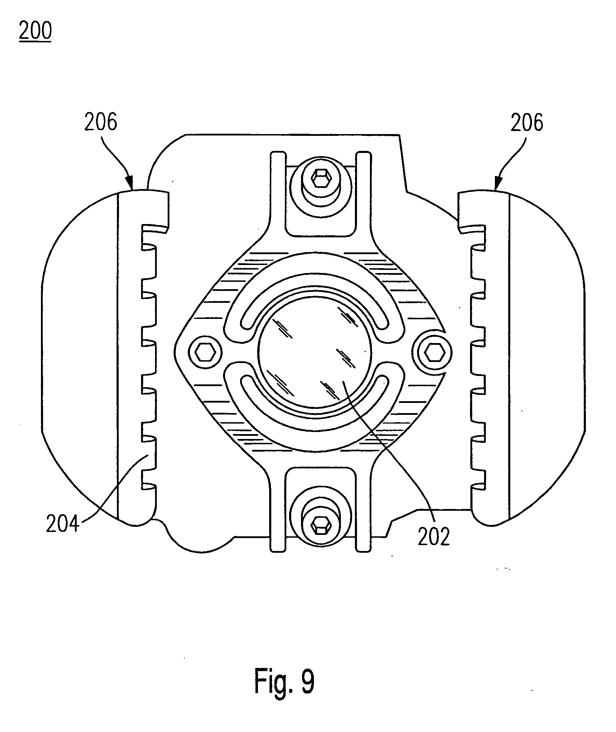

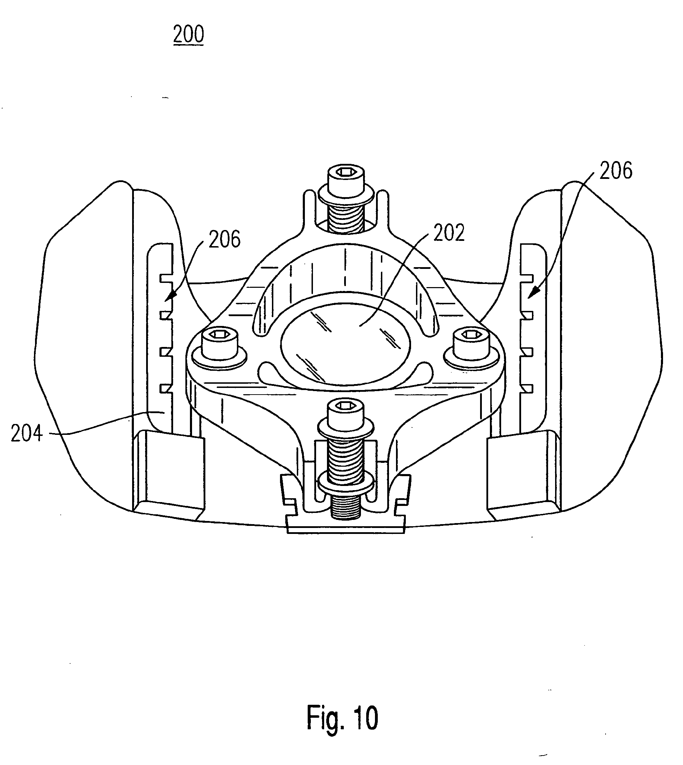

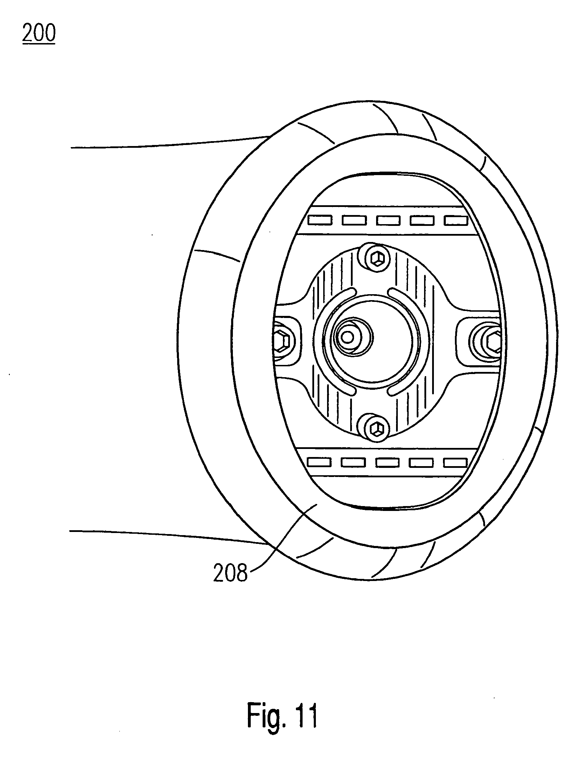

Aus den Figuren 9 bis 11 ist eine erfindungsgemäße Kamera in schematischer Darstellung ersichtlich. Jede punktförmige Lichtquelle 206 ist in diesem Ausführungsbeispiel durch fünf nebeneinander in Reihe angeordnete Laserdioden 204 gebildet, die seitlich neben dem Objektiv 202 der Kamera ausgebildet sind. Es versteht sich, dass anstelle dessen auch eine einzelne Laserdiode 204 realisiert werden kann. Fig. 11 zeigt zusätzlich ein kurzes Abstandsrohr mit einem Ring 208, der für die Anlage an dem Patienten oder dem Patientenzahn geeignet ausgebildet ist und den Abstand zwischen Kamera und Zahn festlegt. Es versteht sich, dass ein derartiger Abstandsring erfindungsgemäß jedoch nicht erforderlich ist.

## Patentansprüche

1. Intraorale Kameraeinrichtung zum Erzeugen eines Bildes eines intraoralen Gegenstandes wie eines Patientenzahnes, die eine Kamera (44) aufweist, die ein Bediener auf den intraoralen Gegenstand richtet und die eine Sichtinformation über den intraoralen Gegenstand liefert, wobei die Kamera (44) eine optische Achse (49) aufweist, die durch ihr Objektiv verläuft, **dadurch gekennzeichnet, dass** die Einrichtung des Weiteren folgendes aufweist:
- eine punktförmige Lichtquelle (48, 50), mit welcher ein begrenzter und im Wesentlichen kreisförmiger Lichtpunkt auf den intraoralen Gegenstand werfbar ist und die derart zu der optischen Achse (49) der Kamera ausgerichtet ist, dass der Auftreffpunkt der optischen Achse (49) mit dem Lichtpunkt zusammenfällt; und
- eine Ausrichteinrichtung (26), die anzeigt, wenn die Kamera (44) im Wesentlichen zum intraoralen Gegenstand ausgerichtet ist, basierend auf der Sichtinformation und dem von der punktförmigen Lichtquelle gelieferten, vom intraoralen Gegenstand abgestrahlten und von der Kamera erfassten Licht;
- wobei ein Ausschnitt des von der Kamera (44) erfassten Bildes in Unterausschnitte (109) aufgeteilt wird, deren Bildinhalte von der Ausrichteinrichtung (26) bewertet werden.

2. Kameraeinrichtung nach Anspruch 1,
**dadurch gekennzeichnet, dass** die Lichtquelle (48, 50) mindestens eine Laserdiode (204) aufweist, die Licht auf den Patientenzahn, wirft, und dass die Kamera (44) eine Sichtinformation über Lichteigenschaften des von der Laserdiode auf dem intraoralen Gegenstand erzeugten Lichtpunkts liefert.

3. Kameraeinrichtung nach einem der vorhergehenden Ansprüche,
**dadurch gekennzeichnet, dass** die optische Achse (49) der Kamera (44) die Mitte des Aüfnahmebereichs der Kamera bildet, und dass die punktförmige Lichtquelle (48, 50) schräg zu der optischen Achse (49) ausgerichtet ist.

4. Kameraeinrichtung nach einem der vorhergehenden Ansprüche,
**dadurch gekennzeichnet, dass** die Schrägstellung der punktförmigen Lichtquelle (48, 50) in solcher Weise vorgesehen ist, dass bei einem vorgegebenen Abstand der Kamera von dem intraoralen Gegenstand, die optische Achse (49) den von der Lichtquelle (48, 50) auf den intraoralen Gegenstand geworfenen Lichtpunkt durchtritt, insbesondere mittig durchtritt.

5. Kameraeinrichtung nach einem der vorhergehenden Ansprüche,
**dadurch gekennzeichnet, dass** die punktförmige Lichtquelle mindestens zwei Laserdioden (204) aufweist, die Licht auf den intraoralen Gegenstand werfen und insbesondere symmetrisch zueinander ausgerichtet sind.

6. Kameraeinrichtung nach einem der vorhergehenden Ansprüche,
**dadurch gekennzeichnet, dass** die Ausrichteinrichtung basierend auf der Sichtformation über Lichteigenschaften eines von den Laserdioden auf dem intraoralen Gegenstand erzeugten Lichtpunkts anzeigt, sobald die Kamera (44) fokussiert hat und/oder sobald sie ausgerichtet ist.

7. Kameraeinrichtung nach einem der vorhergehenden Ansprüche,
**dadurch gekennzeichnet, dass** die Kamera (44) bei der Anzeige, dass die Kamera für eine bestimmte Zeitdauer ausgerichtet ist, manuell oder automatisch zur Aufnahme eines Bildes des intraoralen Gegenstandes betätigbar ist.

8. Kameraeinrichtung nach einem der vorhergehenden Ansprüche,
**dadurch gekennzeichnet, dass** die Ausrichteinrichtung (26) anzeigt, ob die Kamera (44) gegenüber einem ausgewählten Ausschnitt der Sichtinformation ausgerichtet ist, wobei der Ausschnitt das abgestrahlten, von der punktförmigen Lichtquelle stammenden und vom Patientenzahn reflektierende Licht überdeckt.

9. Kameraeinrichtung nach Anspruch 8,
**dadurch gekennzeichnet, dass** der Ausschnitt größer als der Lichtpunkt ist, insbesondere etwa die doppelte Fläche des Lichtpunkts aufweist.

10. Kameraeinrichtung nach Anspruch 6,
**dadurch gekennzeichnet, dass** die Ausrichteinrichtung (26) in ein Raster unterteilbar ist, und dass die Ausrichteinrichtung (26) basierend auf dem Vergleich von Lichteigenschaften in unterschiedlichen Feldern des Rasters anzeigt, ob die Kamera (44) im Wesentlichen ausgerichtet ist.

11. Kameraeinrichtung nach einem der vorhergehenden Ansprüche,
**dadurch gekennzeichnet, dass** die Ausrichteinrichtung (26) einen gegenüber der Sichtinformation des intraoralen Gegenstandes kleineren Ausschnitt festlegt, insbesondere um den Faktor 10 und bevorzugt um den Faktor 100 kleineren Ausschnitt, und dass insbesondere der Ausschnitt in einer punktsymmetrischen Anzahl (9, 25, usw.) von Feldern, in einem vorgegebenen Rastermaß unterteilbar ist, wobei mit der Ausrichteinrichtung vorgegebene Parameter oder Lichteigenschaften von einander gegenüberliegenden Feldern erfassbar und miteinander vergleichbar sind.

12. Verfahren zum Erzeugen eines ausgerichteten Bildes eines intraoralen Gegenstandes, insbesondere eines Patientenzahns, welches das Richten einer Kamera auf den intraoralen Gegenstand zum Liefern von Sichtinformationen über den intraoralen Gegenstand umfasst, wobei das Verfahren des Weiteren die folgenden Schritte aufweist:
- Richten einer punktförmigen Lichtquelle, die einen begrenzten und im Wesentlichen kreisförmigen Lichtpunkt wirft, auf den intraoralen Gegenstand, während der intraorale Gegenstand von der Kamera erfasst wird, wobei die Lichtquelle schräg zu einer durch den Kameraobjektiv verlaufenden optischen Achse ausgerichtet wird; und
- Unterteilung eines von der Kamera erfassten Bildes in Unterabschnitte,
- Bestimmen der Ausrichtung der Kamera durch die Bewertung der Unterabschnitte;
- Anzeigen, dass die Kamera gegenüber dem intraoralen Gegenstand im Wesentlichen ausgerichtet ist, sobald die von der Lichtquelle stammenden und vom intraoralen Gegenstand abgestrahlten Sichtinformationen in der Kamera vorliegen.

13. Verfahren nach Anspruch 12,
**dadurch gekennzeichnet, dass**
- Licht von mindestens einer, insbesondere zwei Laserdiode(n) auf den Patientenzahn gerichtet wird; und
- Sichtinformationen über die Eigenschaften des Lichtes der Laserdioden auf dem intraoralen Gegenstand angezeigt werden.

14. Verfahren nach Anspruch 12 oder 13,
**dadurch gekennzeichnet, dass** es ferner folgende Schritte aufweist:
- Ausrichten der Laserdioden, damit deren Licht auf den intraoralen Gegenstand fällt und Ausrichten der Kamera auf den intraoralen Gegenstand; und
- Anzeigen, sobald die Kamera ausgerichtet ist, basierend auf Sichtinformationen über die Eigenschaften des Lichtes der Laserdioden auf dem Patientenzahn.

15. Verfahren nach einem der Ansprüche 12 bis 14,
**dadurch gekennzeichnet, dass** ein Bild des intraoralen Gegenstandes manuell oder automatisch aufgenommen wird sobald eine Ausrichteinrichtung anzeigt, dass die Kamera für eine gewisse Zeitdauer im Wesentlichen ausgerichtet ist.

16. Verfahren nach einem der Ansprüche 12 bis 15,
**dadurch gekennzeichnet, dass** der Anzeigevorgang bestimmt, ob die Kamera im Wesentlichen ausgerichtet ist, basierend auf einem Ausschnitt der Sichtinformation.

17. Verfahren nach einem der Ansprüche 12 bis 16,
**dadurch gekennzeichnet, dass** der Bestimmungsvorgang folgende Schritte aufweist:
- Unterteilen der Ausrichteinrichtung in einen Raster;
- Anzeigen, ob die Kamera im Wesentlichen ausgerichtet ist;
- Vergleichen der Lichteigenschaften der verschiedenen Felder des Rasters untereinander; und
- Anzeigen, dass die Kamera richtig ausgerichtet ist, durch Markieren des zentralen Feldes der Ausrichteinrichtung.

## Claims

1. An intra-oral camera device for creating an image of an intra-oral subject, such as a patient's tooth, which has a camera (44) which an operator aims at the intra-oral subject and which provides visual information in respect of the intra-oral subject, wherein the camera (44) has an optical axis (49) which extends through its lens; **characterised in that** the device additionally has the following:
- a point light source (48,50), with which a limited and substantially circular light spot can be projected on to the intra-oral subject and which is aligned with respect to the optical axis (49) of the camera in such a way that the point of incidence of the optical axis (49) coincides with the light spot, and
- an aligning device (26) which indicates when the camera (44) is substantially aligned with respect to the intra-oral subject, based on the visual information and on the light provided by the point light source, radiated from the intra-oral subject and captured by the camera;
- wherein one view of the image captured by the camera (44) is subdivided into sub-views, the image contents of which are evaluated by the aligning device (26).

2. A camera device according to Claim 1, **characterised in that** the light source (48,50) has at least one laser diode (204) which projects light on to the patient's tooth, and **in that** the camera (44) provides visual information regarding the light properties of the light spot created on the intra-oral subject by the laser diode.

3. A camera device according to any one of the preceding Claims, **characterised in that** the optical axis (49) of the camera (44) forms the centre of the recording zone of the camera, and **in that** the point light source (48,50) is aligned obliquely relative to the optical axis (49).

4. A camera device according to any one of the preceding Claims, **characterised in that** the oblique position of the point light source (48,50) is provided in such a way that at a predetermined distance of the camera from the intra-oral subject the optical axis (49) passes, in particular passes centrally, through the light spot projected by the light source (48,50) on to the intra-oral subject.

5. A camera device according to any one of the preceding Claims, **characterised in that** the point light source has at least two laser diodes (204) which project light on to the intra-oral subject and, in particular, are aligned symmetrically to one another.

6. A camera device according to any one of the preceding Claims, **characterised in that**, based on the visual information regarding the light properties of a light spot created on the intra-oral subject by the laser diodes, the aligning device indicates as soon as the camera (44) has focussed and/or as soon as it is aligned.

7. A camera device according to any one of the preceding Claims, **characterised in that**, when it is indicated that the camera is aligned for a given period, the camera (44) can be actuated manually or automatically to capture an image of the intra-oral subject.

8. A camera device according to any one of the preceding Claims, **characterised in that** the aligning device (26) indicates whether the camera (44) is aligned with respect to a chosen view of the visual information, in which case the view masks the radiated light originating from the point light source and reflected from the patient's tooth.

9. A camera device according to Claim 8, **characterised in that** the view is larger than the light spot, in particular approximately double the area of the light spot.

10. A camera device according to Claim 6, **characterised in that** the aligning device (26) can be sub-divided into a grid and **in that**, based on a comparison of light properties in different fields of the grid, the aligning device (26) indicates whether the camera (44) is substantially aligned.

11. A camera device according to any one of the preceding Claims, **characterised in that** the aligning device (26) defines a smaller view in relation to the visual information of the intra-oral subject, in particular a view which is smaller by a factor of 10 and preferably by a factor of 100, and **in that** in particular the view can be subdivided into a centro-symmetrical number (9,25,etc.) of fields, in a predetermined grid size, wherein predetermined parameters or light properties of mutually opposing fields can be acquired and compared with one another by the aligning device.

12. A method of creating an aligned image of an intra-oral subject, in particular of a patient's tooth, which comprises aiming the camera at the intra-oral subject so as to provide the visual information regarding the intra-oral subject, wherein the method additionally has the following steps:
- directing a point light source which projects a limited and substantially circular light spot on to the intra-oral subject while the intra-oral subject is recorded by the camera, wherein the light source is aligned obliquely with respect to an optical axis extending through the camera lens, and
- subdividing into sub-views an image captured by the camera,
- determining the alignment of the camera by evaluating the sub-views;
- indicating that the camera is substantially aligned with respect to the intra-oral subject as soon as the visual information, which originates from the light source and is radiated from the intra-oral subject, is present in the camera.

13. A method according to Claim 12, **characterised in that**
- light from at least one, in particular two, laser diode(s) is directed on to the patient's tooth, and
- visual information regarding the properties of the light of the laser diodes on the intra-oral subject is indicated.

14. A method according to Claim 12 or 13, **characterised in that** it further comprises the following steps:
- aligning the laser diodes so that their light is incident upon the intra-oral subject and aligning the camera on the intra-oral subject, and
- indicating as soon as the camera is aligned, based on the visual information regarding the properties of the light of the laser diodes on the patient's tooth.

15. A method according to any one of Claims 12 to 14, **characterised in that** an image of the intra-oral subject is recorded manually or automatically as soon an aligning device indicates that the camera is substantially aligned for a certain period of time.

16. A method according to any one of Claims 12 to 15,
**characterised in that** the indicating device determines whether the camera is substantially aligned, based on one view of the visual information.

17. A method according to any one of Claims 12 to 16, **characterised in that** the determining process has the following steps:
- sub-dividing the aligning device into a grid;
- indicating whether the camera is substantially aligned;
- comparing with one another the light properties of the different fields of the grid, and
- indicating that the camera is correctly aligned by marking the central field of the aligning device.

## Revendications

1. Dispositif de caméra intrabuccale pour générer une image d'un objet intrabuccal, comme une dent d'un patient, qui présente une caméra (44) que l'opérateur dirige sur l'objet intrabuccal et qui procure une information visuelle sur l'objet intrabuccal, où la caméra (44) présente un axe optique (49), qui passe par son objectif, **caractérisé en ce que** le dispositif présente par ailleurs ce qui suit :
- une source lumineuse ponctuelle (48, 50), avec laquelle un point lumineux limité et essentiellement circulaire peut être projeté sur l'objet intrabuccal et qui est dirigé vers l'axe optique (49) de la caméra de sorte que le point d'impact de l'axe optique (49) coïncide avec le point lumineux ; et
- un dispositif d'alignement (26), qui indique lorsque la caméra (44) est essentiellement alignée avec l'objet intrabuccal, sur la base de l'information visuelle et de la lumière produite par la source lumineuse ponctuelle, diffusée par l'objet intrabuccal et détectée par la caméra ;
où un secteur de l'image détectée par la caméra (44) est divisé en sous-secteurs (109), dont le contenu d'image est évalué par le dispositif d'alignement (26).

2. Dispositif de caméra selon la revendication 1, **caractérisé en ce que** la source lumineuse (48, 50) présente au moins une diode laser (204), qui projette la lumière sur la dent du patient, **en ce que** la caméra (44) donne une information visuelle par les propriétés de lumière du point lumineux produit par la diode laser sur l'objet intrabuccal.

3. Dispositif de caméra selon l'une des revendications précédentes, **caractérisé en ce que** l'axe optique (49) de la caméra (44) forme le centre de la zone d'enregistrement de la caméra et **en ce que** la source lumineuse ponctuelle (48, 50) est dirigée en oblique par rapport à l'axe optique (49).

4. Dispositif de caméra selon l'une des revendications précédentes, **caractérisé en ce que** la position oblique de la source lumineuse ponctuelle (48, 50) est prévue de telle sorte que pour une distance donnée de la caméra à l'objet intrabuccal, l'axe optique (49) traverse le point lumineux projeté sur l'objet intrabuccal par la source lumineuse (48, 50), en particulier le traverse centralement.

5. Dispositif de caméra selon l'une des revendications précédentes, **caractérisé en ce que** la source lumineuse ponctuelle présente au moins deux diodes laser (204), qui projettent la lumière sur l'objet intrabuccal et sont dirigées en particulier de manière symétrique l'une par rapport à l'autre.

6. Dispositif de caméra selon l'une des revendications précédentes, **caractérisé en ce que** le dispositif d'alignement indique, sur la ase de l'information visuelle, par les propriétés de lumière d'un point lumineux engendré sur l'objet intrabuccal par les diodes laser, dès que la caméra (44) est focalisée et/ou dès qu'elle est alignée.

7. Dispositif de caméra selon l'une des revendications précédentes, **caractérisé en ce que** la caméra (44) est actionnable manuellement ou automatiquement pour l'enregistrement d'une image de l'objet intrabuccal, lors de l'indication que la caméra est alignée pendant une certaine période.

8. Dispositif de caméra selon l'une des revendications précédentes, **caractérisé en ce que** le dispositif d'alignement (26) indique si la caméra (44) est alignée avec un secteur choisi de l'information visuelle, où le secteur couvre la lumière diffusée, provenant de la source lumineuse ponctuelle et réfléchie sur la dent du patient.

9. Dispositif de caméra selon la revendication 8, **caractérisé en ce que** le secteur est plus grand que le point lumineux, en particulier environ une surface double du point lumineux.

10. Dispositif de caméra selon la revendication 6, **caractérisé en ce que** le dispositif d'alignement (26) peut être divisé en une grille et **en ce que** le dispositif d'alignement (26) indique, sur base de la comparaison des propriétés de lumière dans différents champs de la grille, si la caméra (44) est essentiellement alignée.

11. Dispositif de caméra selon l'une des revendications précédentes, **caractérisé en ce que** le dispositif d'alignement (26) détermine un secteur plus petit par rapport à l'information visuelle de l'objet intrabuccal, en particulier un secteur plus petit d'un facteur 10 et de préférence d'un facteur 100, et **en ce qu'**en particulier, le secteur peut être divisé en un nombre à symétrie ponctuelle (9, 25, etc.) de champs, dans une dimension donnée de la grille, où avec le dispositif d'alignement, des paramètres donnés ou des propriétés de la lumière de champs opposés l'un par rapport à l'autre, peuvent être saisis et comparés l'un avec l'autre.

12. Procédé pour générer une image alignée d'un objet intrabuccal, en particulier une dent d'un patient, qui comprend l'orientation d'une caméra sur l'objet intrabuccal pour procurer des informations visuelles de l'objet intrabuccal, où le procédé présente par ailleurs, les étapes suivantes :
- l'orientation d'une source lumineuse ponctuelle, qui projette un point lumineux limité et essentiellement circulaire sur l'objet intrabuccal, pendant que l'objet intrabuccal est saisi par la caméra, où la source lumineuse est alignée en oblique à l'axe optique développé par la l'objectif de la caméra ; et
- division d'une image saisie par la caméra en sous-secteurs ;
- détermination de l'alignement de la caméra par l'évaluation des sous-secteurs ;
- indication du fait que la caméra est alignée essentiellement avec l'objet intrabuccal, dès que les informations visuelles provenant de la source lumineuse et diffusées par l'objet intrabuccal sont présentes dans la caméra.

13. Procédé selon la revendication 12, **caractérisé en ce que**
- la lumière d'au moins une, en particulier deux diodes laser est orientée sur la dent du patient, et
- les informations visuelles sont indiquées par les propriétés de la lumière des diodes laser sur l'objet intrabuccal.

14. Procédé selon la revendication 12 ou 13, **caractérisé en ce qu'**il présente en outre les étapes suivantes :
- alignement des diodes laser pour que leur lumière arrive sur l'objet intrabuccal et alignement de la caméra sur l'objet intrabuccal, et
- indication du moment où la caméra est alignée, sur la base des informations visuelles par les propriétés de la lumière des diodes laser sur la dent du patient.

15. Procédé selon l'une des revendications 12 à 14, **caractérisé en ce qu'**une image de l'objet intrabuccal est relevée manuellement ou automatiquement, dès que la caméra est essentiellement alignée pendant une certaine période.

16. Procédé selon l'une des revendications 12 à 15, **caractérisé en ce que** le dispositif d'alignement détermine si la caméra est essentiellement alignée, sur la base d'un secteur de l'information visuelle.

17. Procédé selon l'une des revendications 12 à 16, **caractérisé en ce que** le processus de détermination présente les étapes suivantes :
- division du dispositif d'alignement en une grille ;
- indication si la caméra est essentiellement alignée ;
- comparaison des propriétés de lumière des différents champs de la grille, les uns avec les autres ; et
- affichage de ce que la caméra est convenablement alignée, par marquage du champ central du dispositif d'alignement.
